# EUROPEAN PATENT SPECIFICATION

(11) **EP 3 693 971 B1**
(45) Date of publication and mention of the grant of the patent: **03.12.2025**
(21) Application number: 19156086.1
(22) Date of filing: 07.02.2019
(51) Int. Cl.: G16B 30/20

(54) **BIOLOGICAL SEQUENCING**
BIOLOGISCHE SEQUENZIERUNG
SÉQUENÇAGE BIOLOGIQUE

(43) Date of publication of application: 12.08.2020
(73) Proprietor: NV Bioclue, 3930 Hamont (BE)
(72) Inventor: Van Hyfte, Dirk, 3930 Hamont (BE); Brands, Ingrid, 3930 Hamont (BE)
(74) Representative: Winger

(56) References cited:
- ANONYMOUS: "De novo peptide sequencing - Wikipedia", 9 January 2019 (2019-01-09), XP055607198, Retrieved from the Internet <URL:https://en.wikipedia.org/w/index.php?title=De_novo_peptide_sequencing&oldid=877544803> [retrieved on 20190719]
- W. NELSON ET AL: "Integrating sequence with FPC fingerprint maps", NUCLEIC ACIDS RESEARCH, vol. 37, no. 5, 9 January 2009 (2009-01-09), pages e36 - e36, XP055607919, ISSN: 0305-1048, DOI: 10.1093/nar/gkp034

## Description

### Technical field of the invention

The present invention relates to the handling of biological sequence information, and more particularly to generating said biological sequence information, e.g. by sequencing and sequence assembly. More specifically, it relates to sequencing a biopolymer or biopolymer fragment taking into account information contained in a repository of fingerprint data strings.

### Background of the invention

Biological sequencing has evolved at a blinding speed in the last decades, enabling along the way the human genome project which achieved a complete sequencing of the human genome already more than 15 years ago. To fuel this evolution, ample technical progress has been required, spanning from advances in sample preparation and sequencing methods to data acquisition, processing and analysis. Concurrently, new scientific fields have spawned and developed, including genomics, proteomics and bioinformatics.

Fuelled by the postgenomic era's emphasis on data acquisition, this evolution has resulted in the accumulation of enormous amounts of sequence data. However, the ability to organize, analyse and interpret this sequence, to extract therefrom biologically relevant information, has been trailing behind. This problem is further compounded by the magnitude of new sequence information which is still generated on a daily basis. Muir et al. observed that this is sparking a paradigm shift and have commented on the resulting changing cost structure for sequencing and other associated hurdles (MUIR, Paul, et al. The real cost of sequencing: scaling computation to keep pace with data generation. Genome biology, 2016, 17.1: 53.).

Currently, the sequencing methods most often employed are those of the so called 'high-throughput' or 'next generation sequencing' (NGS). In contrast to the first generation of sequencing, NGS is typically characterized by being highly scalable, allowing an entire genome to be sequenced at once. Typically, this is accomplished by fragmenting a larger sequence into smaller fragments, randomly sampling for a fragment, and sequencing it. After sequencing the different fragments, the original sequence can be reconstructed using sequence assembly, in which the sequence fragments are aligned and merged on the basis of their overlapping regions.

However, sequencers are not flawless and sequencing errors (such as insertions, substitutions and deletions) can always occur, particularly when a high throughput is sought. If the sequence fragments to be assembled contain errors, this obviously complicates the reconstruction of the original sequence as corresponding areas may no longer overlap. Furthermore, also the errors may propagate into the final sequence, e.g. resulting in mistaken variant calling. Some strategies have been developed for dealing with these sequencing errors, such as disclosed by Shmilovici et al. (SHMILOVICI, Armin; BEN-GAL, Irad. Using a VOM model for reconstructing potential coding regions in EST sequences. Computational Statistics, 2007, 22.1: 49-69.). However, no efficient method is currently known to validate directly whether a (fragment) sequence is correct or whether it contains one or more sequence errors.

De novo peptide sequencing in general has been described at https://en. wikipedia.org/w/index.php?title=De novo peptide sequencing&oldid=877544803.

Nelson and Soderlund (Nelson, W., & Soderlund, C. (2009). Integrating sequence with FPC fingerprint maps. Nucleic Acids Research, 37(5), e36-e36.) discussed the FPC (fingerprinted contigs) software package containing three modules that maximize the value of a bacterial artificial clone (BAC) fingerprint map, BAC end sequences (BESs) and draft genomic sequence: the blast some sequence (BSS) module, minimal tiling path (MTP) module and draft sequence integration (DSI) module.

There is thus still a need in the art for further improvements in sequencing and sequence assembly.

### Summary of the invention

The invention is defined by the appended claims.

It is an object of the present invention to provide a good way to generate biological sequence information. This objective is accomplished by methods, devices and data structures according to the present invention.

The claimed invention is set out in the appended set of claims.

It is an advantage of embodiments that a repository of fingerprint data strings corresponding to characteristic biological subsequences can be provided. It is a further advantage of embodiments that the biological subsequences need not be of a single length, as is the case for e.g. k-mers.

It is an advantage of embodiments that further data can be included in the repository, such as data on the unit(s) which may succeed or precede a characteristic biological subsequence, data on the secondary/tertiary/quaternary structure of a characteristic biological subsequence, data on a relationship between fingerprints, etc.

It is an advantage of embodiments that the sequencing of biopolymers and biopolymer fragments can be improved (e.g. by decreasing the likelihood of errors or by speeding up the process) by relying on information contained in the repository of fingerprint data strings.

It is an advantage of embodiments that a provisionally suggested biological sequence can be validated or rejected. It is an advantage of embodiments that errors occurring during sequencing can be reduced.

It is an advantage of embodiments that the speed of sequencing can be improved by predicting the next unit in the sequence or by limiting the number of options therefor.

It is an advantage of embodiments that a biological sequence can be relatively easily and efficiently processed. It is a further advantage of embodiments that a biological sequence can be analysed in a lexical or even a semantic fashion.

It is an advantage of embodiments that the processed biological sequence can be constructed by replacing therein the identified characteristic biological subsequences by markers associated with the corresponding fingerprint data strings.

It is an advantage of embodiments that the portions of the biological sequence which do not correspond to one of the characteristic biological subsequences can be handled in a variety of ways. It is a further advantage of some embodiments that the biological sequence can be processed in a completely lossless way (i.e. no information is lost by processing). It is a further advantage of alternative embodiments that the biological sequence can be processed in a way that the more important information is distilled in a more condensed format.

It is an advantage that the processed biological sequences may be compressed so that they take up less storage space than their unprocessed counterparts.

It is an advantage of embodiments that that matching portions of the biological sequence to the characteristic biological subsequences is not solely limited to the primary structure, but can also take into account the secondary/tertiary/quaternary structure.

It is an advantage of embodiments that a secondary/tertiary/quaternary structure of a biological subsequence can be at least partially elucidated based on the known secondary/tertiary/quaternary structure of characteristic biological subsequences contained therein. It is a further advantage of embodiments that biological sequence design (e.g. protein) design can be assisted or facilitated.

It is an advantage of embodiments that a repository of processed biological sequence can be constructed and stored.

It is an advantage that the repository of processed biological sequences can be quickly searched and navigated. It is a further advantage that the storage size of the repository may be relatively small, compared to the known databases, by populating it with compressed processed biological sequences.

It is an advantage of embodiments that the comparison of biological sequences can be changed from an NP-complete or NP-hard problem to a polynomial-time problem. It is a further advantage of embodiments that comparison can be performed in a greatly reduced time and scales well with increasing complexity (e.g. increasing length of or number of biological sequences). It is yet a further advantage of embodiments that the required computational power and storage space can be reduced.

It is an advantage of embodiments that a degree of similarity can be calculated between biological sequences. It is a further advantage of embodiments that a plurality of biological sequences can be ranked based on their degree of similarity.

It is an advantage of embodiments that a sequence similarity search can be quickly and easily performed (e.g. in polynomial time).

It is an advantage of embodiments that compared biological sequences can be easily and quickly aligned (e.g. in polynomial time).

It is an advantage of embodiments that also a plurality of sequences can be easily and quickly compared and aligned. It is a further advantage of embodiments that there is no accumulation of errors during the alignment, as is the case in currently known methods (e.g. based on progressive alignment).

It is an advantage of embodiments that sequences of biopolymer fragments can be easily and quickly aligned and merged to reconstruct the original biopolymer sequence.

It is an advantage of embodiments that the methods may be implemented by a variety of systems and devices, such as computer-based systems or a sequencer, depending on the application. It is a further advantage of embodiments that the methods can be implemented by a computer-based system, including a cloud-based system.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Figs. 1 and 2 are graphs showing expected progress enabled by embodiments of the present invention.
Figs. 3 to 6 are diagrams depicting systems in accordance with embodiments of the present invention.
Figs. 7 and 10 are graphs comparing the total length of search results using, on the one hand, a prior art method (dotted line) and, on the other hand, a method using characteristic biological subsequences (solid line).
Figs. 8 and 11 are graphs comparing the Levenshtein distance of search results using, on the one hand, a prior art method (dotted line) and, on the other hand, a method using characteristic biological subsequences (solid line).
Figs. 9 and 12 are graphs comparing the longest common substring of search results using, on the one hand, a prior art method (dotted line) and, on the other hand, a method using characteristic biological subsequences (solid line).

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms before, after, and the like in the description and the claims are used for descriptive purposes and not necessarily for describing relative positions. It is to be understood that the terms so used are interchangeable with their antonyms under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. The term "comprising" therefore covers the situation where only the stated features are present and the situation where these features and one or more other features are present. Thus, the scope of the expression "a device comprising means A and B" should not be interpreted as being limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment. The claims following the detailed description are hereby expressly incorporated into this detailed description, with each claim standing on its own as a separate embodiment of this invention.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practised without these specific details. In other instances, well-known methods, structures and techniques have not been shown in detail in order not to obscure an understanding of this description.

The following terms are provided solely to aid in the understanding of the invention.

As used herein, a biological sequence is a sequence of a biopolymer defining at least the biopolymer's primary structure. The biopolymer can for example be a deoxyribonucleic acid (DNA), ribonucleic acid (RNA) or a protein. The biopolymer is typically a polymer of biomonomers (e.g. nucleotides or amino acids), but may in some instances further include one or more synthetic monomers.

As used herein, a 'unit' in a biological sequence is an amino acid when the biological sequence relates to a protein and is a codon when the biological sequence relates to DNA or RNA.

As used herein, a biological subsequence is a portion of a biological sequence, smaller than the full biological sequence. The biological subsequence may, for example, have a total length of 100 units or less, preferably 50 or less, yet more preferably 20 or less.

As used herein, some concepts will be illustrated with examples relating to proteins and it will be assumed that the possible monomeric units are the 20 canonical (or 'standard') amino acids. However, it is clear that this is merely to simplify the illustration and that similar embodiments can likewise be formulated with an extended number of amino acids (e.g. adding non-canonical amino acids or even synthetic compounds), or relating to DNA or RNA. In the case of DNA or RNA, a link between the DNA or RNA and proteins can be easily made through the correspondence between codons and amino acids.

As used herein, 'secondary/tertiary/quaternary' refers to 'secondary and/or tertiary and/or quaternary'.

It was surprisingly realized within the present invention that, where it was previously assumed that the primary structure of a biological sequence consists of an essentially independent selection of units, so that there are e.g. mⁿ biological sequences of length n based on m possible units (e.g. 20ⁿ based on 20 canonical amino acids), this in fact not observed in nature. Indeed, it was discovered that from a certain length onwards, not every theoretical combination is seen. To give but one example: the protein subsequence 'MCMHNQA' is not found in any protein in the public databases. It has been contemplated that this is not a mere hiatus in the databases, but that this absence has a physical and/or chemical origin. Without being bound by theory, to name but one possible effect, the steric hindrance of the neighbouring amino acids (e.g. 'MCMHNQ' in the above example) may prohibit one or more other amino acids (e.g. 'A' in the above example) from binding thereto. As such, once an absent subsequence has been identified, computational studies can be used to validate whether this subsequence could potentially occur or whether its existence is physically impossible (or improbable, e.g. because it's chemically unstable). The 'certain length' referred to above depends on the data set that is being considered, but e.g. corresponds to about 5 or 6 amino acids for the publicly available protein sequence databases (which substantially reflect the total diversity seen in nature). For a more limited set (e.g. a set filtered based on a particular criterion), less than the theoretical maximum of mⁿ combinations is already found for a length of about 4 or 5.

Simultaneously, because the subsequence 'MCMHNQA' does not exist, the subsequence 'MCMHNQ' is not merely a random combination of 5 amino acids but gains additional significance; such subsequences will be further referred to as 'characteristic biological subsequences' or 'fingerprints'. Because of the added significance or meaning of these fingerprints, it can be considered that the present invention handles biological sequence information in a more semantic fashion. In general, a characteristic subsequence is characterized by having for the unit directly following (or preceding) it less possible options than the maximum number of units (e.g. less than the 20 canonical amino acids); in other words, at least one of the units cannot follow (or precede) it. However, it is possible to select a stricter definition: e.g. only those subsequences which have 15 units or fewer which can possibly follow it, or 10 or fewer, 5 or fewer, 3, 2 or even 1. Furthermore, it can be chosen to consider each such subsequence as a fingerprint, or to consider only those subsequences as fingerprints which do not already comprise another fingerprint. For example: taking 'MCMHNQ' as a fingerprint, there will be longer subsequences which comprise 'MCMHNQ' and which also have less than the theoretical number of units which can follow (or precede) it; in that case, there is the option to consider both the longer subsequences and 'MCMHNQ' as fingerprints, or to consider only 'MCMHNQ' as a fingerprint.

It was then surprisingly found that a limited set of characteristic biological subsequences can be identified. Furthermore, it was observed that these characteristic biological subsequences strike a balance between, on the one hand, being sufficiently specific so that not every characteristic biological subsequence is found in every biological sequence and, on the other hand, being common enough that the known biological sequences typically comprise at least one of these fingerprints.

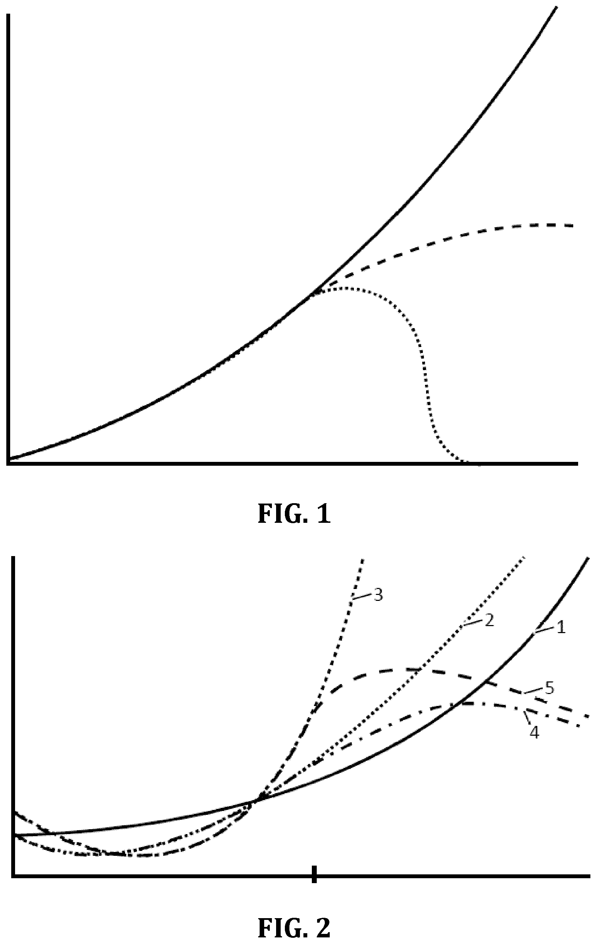

Based on this discovery, new approaches to handling biological sequence information, in all its different but interrelated stages, can be formulated. These approaches can be considered as being akin to a more lexical analysis of the sequences. The result is schematically depicted in Fig. 1, which shows the complexity scaling of the biological sequence information with an increasing number of units (n). This complexity may be the total number of possible combinations of units, but that in turn also relates to the computational effort (e.g. time and memory) needed for handling it (e.g. for performing a similarity search). The solid curve depicts the number of theoretical combinations assuming all units are selected independently, scaling as mⁿ, which also corresponds to the scaling of the currently known algorithms. The dashed curve depicts the number of actual combinations found in nature (as observed within the present invention), where the curve departs from mⁿ at around 5 or 6 units and asymptotically flattens off for high n. The dotted line shows the number of sequences which correspond for the first time to a characteristic sequence for which the number of units which can follow it is equal to 1; here 'for the first time' means that longer sequences are never counted if they comprise an already counted fingerprint. Thus, the latter corresponds to the number of fingerprints of length n (as observed within the present invention), when the definition thereof is selected as a subsequence which has only 1 unit which can possibly follow it and which does not already comprise another (shorter) fingerprint (cf. supra).

Fig. 2 depicts the predicted benefits of the present invention in time, where the mark on the bottom axis depicts the present day. Curve 1 shows Moore's law as a reference. Curve 2 shows the total amount of acquired sequencing data. Curve 3 shows the total cost of processing and maintaining said sequencing data. By handling biological sequence information as proposed in the present invention, the total required storage for sequencing data and the total cost of data processing and maintenance are expected to drop as depicted in curves 4 and 5 respectively.

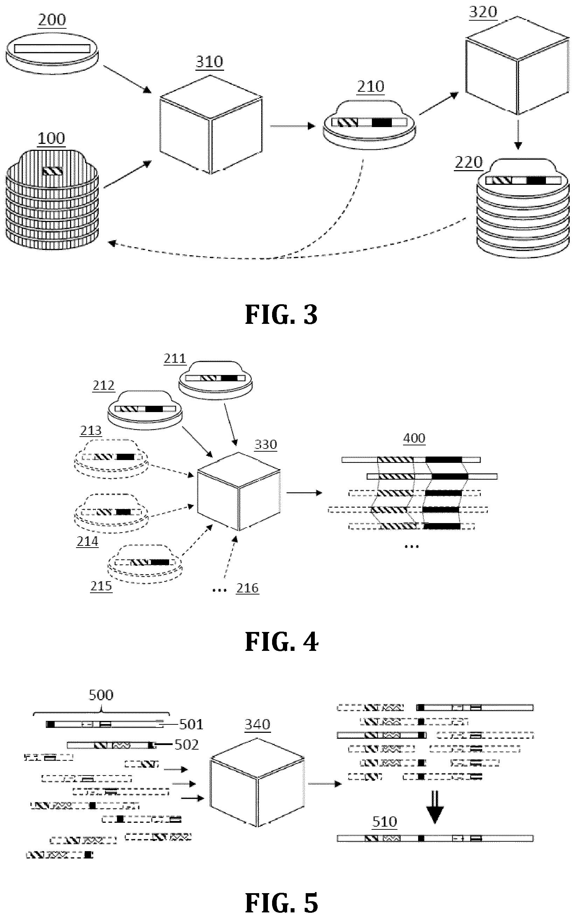

In a first aspect-used in the claimed invention-, a repository of fingerprint data strings is described, each fingerprint data string representing a characteristic biological subsequence, the repository comprising at least: a first fingerprint data string representing a first characteristic biological subsequence of a first length, a second fingerprint data string representing a second characteristic biological subsequence of a second length, and, for at least one of the fingerprint data strings, data related to one or more units which can appear (e.g. which can realistically appear, such as those combinations which are stable) directly before or after the characteristic biological subsequence when said characteristic biological subsequence is present in a biological sequence; wherein the first and the second length are equal to 4 or more and wherein the first and the second length differ from one another. A repository (e.g. database) of fingerprint data strings 100 is schematically depicted in Figs. 3 and 4, which will be discussed in more detail under the second, third and fifth aspect.

In embodiments, the length may correspond to the number of units. In embodiments, the length may be up to 100 or less, preferably 50 or less, yet more preferably 20 or less. In embodiments, the first and the second length may be equal to 5 or more, preferably 6 or more. In embodiments, the first and second characteristic biological subsequences may have a length between 4 and 20, preferably 5 and 15, yet more preferably 6 and 12.

In embodiments, the repository of fingerprint data strings may comprise at least 3 fingerprint data strings which differ in length from one another, preferably at least 4, yet more preferably at least 5, most preferably at least 6. Since the characteristic biological subsequences are not defined by their length, but by the number of possible units which follow (or precede) it, a set of characteristic biological subsequences typically advantageously comprises subsequences of varying lengths. The repository of fingerprint data strings in the present invention differs from e.g. a collection of k-mers (as is known in the art) in that it comprises biological subsequences of varying lengths. Furthermore, a collection of k-mers typically comprises every permutation (i.e. every possible combination of units) of length k; this is not the case for the present repository of fingerprint data strings.

In embodiments, the fingerprint data strings may be protein fingerprint data strings, DNA fingerprint data strings or RNA fingerprint data strings. In embodiments, the characteristic biological subsequence may be a characteristic protein subsequence, a characteristic DNA subsequence or a characteristic RNA subsequence. In embodiments, the repository of fingerprint data strings may comprise (e.g consist of) protein fingerprint data strings, DNA fingerprint data strings, RNA fingerprint data strings or a combination of one or more of these. A characteristic protein subsequence can in embodiments be translated into a characteristic DNA or RNA subsequence, and vice versa. This translation can be based on the well-known DNA and RNA codon tables. Similarly, a protein fingerprint data string can be translated into a DNA or RNA fingerprint data string. In embodiments, a repository of DNA or RNA fingerprint data strings may comprise information on equivalent codons (i.e. codons which code for the same amino acid). This information on equivalent codons can be included in the fingerprint data string as such, or stored separately therefrom in the repository.

In embodiments, the data related to the one or more units which can appear directly before or after the characteristic biological subsequence may comprise the number of possible units, the possible units as such, the likelihood (e.g. probability) for each unit, etc.

In embodiments, the repository of fingerprint data strings may further comprise additional data for at least one of the fingerprint data strings. In preferred embodiments, said data may be included in the fingerprint data string. In alternative embodiments, said data may be stored separately from the fingerprint data strings.

In embodiments, the additional data may comprise data related to a secondary/tertiary/quaternary structure of the characteristic biological subsequence when said characteristic biological subsequence is present in a biopolymer. In embodiments, the data related to the secondary (or tertiary/quaternary) structure may comprise the number of possible structures, the possible structures as such, the likelihood (e.g. probability) for each structure, etc. In the case of multiple possible secondary/tertiary/quaternary structures for a given characteristic biological subsequence, the repository may in embodiments comprise a separate entry for each combination of the characteristic biological subsequence and an associated secondary/tertiary/tertiary structure. In alternative embodiments, the repository may comprise one entry comprising the characteristic biological subsequence and a plurality of its associated secondary/tertiary/quaternary structures. In embodiments, the secondary/tertiary/quaternary structure may be more relevant for proteins than for DNA and RNA.

In embodiments, the additional data may comprise data related to a relationship between the characteristic biological subsequence and one or more further characteristic biological subsequences. In embodiments, the data related to a relationship between the characteristic biological subsequence may comprise further characteristic biological subsequences which commonly appear in its vicinity, the likelihood for the further characteristic biological subsequence to appear in its vicinity, a particular significance (e.g. a biologically relevant meaning, such as a trait or a secondary/tertiary/quaternary structure) of these characteristic biological subsequences appearing close to one another, etc. In embodiments, the relationship may be expressed in the form of a path between two or more characteristic biological subsequences and may include the order of the characteristic biological subsequences, their inter-distance, etc. In embodiments, the additional data may also comprise metadata useful for building said paths.

In some embodiments, the additional data may have been retrieved from a known data set; e.g. the secondary/tertiary/quaternary structure of several biological sequences is available in the art. In other embodiments, the additional data may have been may be extracted from a processed biological sequence as defined below or from a repository of processed biological sequences as defined below. For example, after processing a biological sequence as defined below (or building a repository of processed biological sequences as defined below), relationships between the characteristic biological subsequences (e.g. paths) may be extracted and added to a repository of fingerprint data strings as described above; this is schematically depicted in Fig. 4 by the dashed arrows pointing from the processed biological sequence 210 and the repository of processed biological sequences 220 to the repository of fingerprint data strings 100.

In a second aspect-in accordance with the claimed invention-, a method for sequencing a biopolymer or biopolymer fragment is described, comprising sequencing the biopolymer or biopolymer fragment taking into account information contained in a repository of fingerprint data strings as defined in any embodiments of the first aspect. Fig. 3 schematically shows a sequencer 350 which sequences biopolymer (fragments) 500 using information contained in the repository of fingerprint data strings 100.

In embodiments, the method may comprise sequencing an initial (e.g. provisional or partial) biological sequence.

In one alternative, taking into account information contained in the repository of fingerprint data strings comprises searching a provisional biological sequence for occurrences of a characteristic biological subsequence represented by one of the fingerprint data strings, and subsequently validating or rejecting the provisional biological sequence by, for each occurrence, determining whether or not a unit appearing directly before or after the characteristic biological subsequence conforms with the data as defined in embodiments of the first aspect. Since the repository can contain data on the units which can appear before or after a fingerprint, this information can be advantageously used to verify whether a provisional biological sequence is agreement therewith. If it is not, the provisional biological sequence can be rejected and redone.

In another-optionally complementary-alternative, taking into account information contained in the repository of fingerprint data strings comprises searching a head or a tail of a partial biological sequence for occurrences of a characteristic biological subsequence represented by one of the fingerprint data strings and subsequently predicting a monomer appearing respectively directly before or after of the characteristic biological subsequence from the data as defined in embodiments of the first aspect. Since the repository can contain data on units which are the only possible option for appearing before or after a particular fingerprint, this information can be advantageously used to speed up the sequencing by directly appending that unit to the partial sequence; thereby allowing the actual sequencing to skip past said unit. In embodiments, the repository may contain data on a series of two, three, or more units which together are the only possible option for appearing before or after a particular fingerprint. In this case, the whole series can be advantageously directly appended to the partial sequence; thereby allowing the actual sequencing to skip past these units. Similarly, if the repository indicates that for the observed fingerprint a limited number (but more than 1) of options are possible as further units (e.g. two or three options), this information can still allow the sequencer to more quickly identify the specific unit in the present instance.

In embodiments, the searching may be as described for step b of the third aspect.

In a third aspect-considered useful for understanding the claimed invention-, a method for processing a biological sequence is described, comprising: (a) retrieving one or more fingerprint data strings from the repository as defined in any embodiment of the first aspect, (b) searching the biological sequence for occurrences of the characteristic biological subsequences represented by the one or more fingerprint data strings, and (c) constructing a processed biological sequence comprising for each occurrence in step b a fingerprint marker associated with the fingerprint data string which represents the occurring characteristic biological subsequence. Fig. 4 schematically shows a sequence processing unit 310 which processes a biological sequence 200 using a repository of fingerprint data strings 100, thereby obtaining a processed biological sequence 210.

In embodiments, the biological sequence to be processed may be biological sequence of a biopolymer fragment, obtainable by the method for sequencing according to the second aspect.

In some embodiments, the marker may be a reference string. Such a reference string may for example point towards the corresponding fingerprint data string in the repository. In other embodiments, the marker may be the fingerprint data string as such, or a portion thereof.

In embodiments, the biological sequence may comprise: (i) one or more first portions, each first portion corresponding to one of the characteristic biological subsequences represented by the one or more fingerprint data strings, and (ii) one or more second portions, each second portion not corresponding to any of the characteristic biological subsequences represented by the one or more fingerprint data strings. In embodiments, constructing the processed biological sequence in step c may comprise replacing at least one first portion by the corresponding marker. In embodiments, constructing the processed biological sequence in step c may further comprise adding positional information about said first portion to the processed biological sequence (e.g. appended to the marker). In embodiments, constructing the processed biological sequence in step c may comprise leaving at least one second portion unchanged, and/or replacing at least one second portion by an indication of the length of said second portion, and/or entirely removing at least one second portion. When leaving the second portions unchanged, the biological sequence is able to be processed in a completely lossless way.

In embodiments, the processed biological sequence can be formulated in a condensed format. For example, by replacing the characteristic biological subsequences (i.e. first portions) with reference strings and/or by replacing the second portions with either an indication of its length or entirely removing it, a processed biological sequence is obtained which requires less storage space than the original (i.e. unprocessed) biological sequence. Additional data compression can be achieved by making use of paths which can represent multiple fingerprints by their interrelation.

In embodiments, the one or more fingerprint data strings may be in a different biological format than the biological sequences (e.g. protein vs DNA vs RNA sequence information) and step b may further comprise translating or transcribing the characteristic biological subsequences prior to the searching.

In embodiments, the searching in step b may include searching for a partial match or an equivalent match (e.g. an equivalent codon, or a different amino acid resulting in the same secondary/tertiary/quaternary structure). In embodiments, the searching in step b may take into account a secondary/tertiary/quaternary structure of the characteristic biological subsequence. The secondary, tertiary and quaternary are typically more evolutionary conserved and often variation in the primary structure occur which do not change the function of the biopolymer, e.g. because the secondary/tertiary/quaternary structure of its active sites is substantially conserved. The secondary/tertiary/quaternary structure may therefore reveal relevant information about the biopolymer which would be lost when strictly searching for a fully matching primary structure.

In embodiments, the method may comprise a further step d, after step c, of at least partially inferring a secondary/tertiary/quaternary structure of the processed biological subsequence based on the data related to the secondary/tertiary/quaternary structure as defined in embodiments of the first aspect. This at least partial elucidation of the secondary/tertiary/quaternary structure can help to assist and/or facilitate biological sequence design. In embodiments wherein a single primary structure of a characteristic biological subsequence is linked to a plurality of secondary or tertiary or quaternary structures, the secondary/tertiary/quaternary structure may be disambiguated based on the context in which the characteristic biological subsequence is found, such as the characteristic biological subsequences which it is surrounded by. The information needed for such disambiguation may, for example, be found in the repository of fingerprint data strings in the form of data related to a relationship in terms secondary/tertiary/quaternary structure of between the characteristic biological subsequence and one or more further characteristic biological subsequences, as defined in embodiments of the first aspect.

In a fourth aspect-considered useful for understanding the claimed invention-, a processed biological sequence is described, obtainable by the method according to any embodiment of the third aspect. A processed biological sequence 210 is schematically depicted in Fig. 4.

In a fifth aspect-considered useful for understanding the claimed invention-, a method for building a repository of processed biological sequences is described, comprising populating said repository with processed biological sequences as defined any embodiment of the fourth aspect. Fig. 4 schematically shows a repository building unit 320 storing a processed biological sequence 210 into a repository of processed biological sequences 220.

In a sixth aspect-considered useful for understanding the claimed invention-, a repository of processed biological sequences is described, obtainable by the method according to any embodiment of the fifth aspect. A repository of 220 is schematically depicted in Fig. 4.

In embodiments, the repository may be a repository of processed biological fragment sequences (i.e. processed biological sequences of biopolymer fragments).

In embodiments, the repository may be a database. In some embodiments, the repository of processed biological sequences may be an indexed repository. The repository may, for example, be indexed based on the fingerprint markers (corresponding to the characteristic biological subsequences) present in each processed biological sequence. In other embodiments, the repository may be a graph repository.

In a seventh aspect-considered useful for understanding the claimed invention-, a method for comparing a first biological sequence to a second biological sequence is described, comprising: (a) processing the first biological sequence by the method according to any embodiment of the third aspect to obtain a first processed biological sequence, or retrieving the first processed biological sequence from a repository as defined in any embodiment of the sixth aspect, (b) processing the second biological sequence by the method according to any embodiment of the third aspect to obtain a second processed biological sequence, or retrieving the second processed biological sequence from a repository as defined in any embodiment of the sixth aspect, and (c) comparing at least the fingerprint markers in the first processed biological sequence with the fingerprint markers in the second processed biological sequence. Fig. 5 schematically shows a comparison unit 330 comparing at least a first biological sequence 211 and a second biological sequence 212 to output results 400.

By using characteristic biological subsequences according to embodiments(through the fingerprint markers in the processed biological sequences), the problem of comparing sequences is advantageously reformulated from an NP-complete or NP-hard problem to a polynomial-time problem. Indeed, identifying the fingerprints in a sequence and subsequently comparing sequences based on these fingerprints, which can be considered as a lexical approach, is computationally much simpler than the currently used algorithms (which e.g. compare full sequences based on a sliding windows approach). The comparison can therefore be performed markedly faster and furthermore scales well with increasing complexity (e.g. increasing length of or number of biological sequences), even while requiring less computation power and storage space.

In embodiments, the second biological sequence may be a reference sequence.

In embodiments, step c may comprise identifying whether one or more characteristic biological subsequences (represented by the fingerprint markers) in the first processed biological sequence correspond (e.g. match) with one or more characteristic biological subsequences (represented by the fingerprint markers) in the second processed biological sequence. In embodiments, step c may comprise identifying whether the corresponding characteristic biological subsequences appear in the same order in the first processed biological sequence as in the second processed biological sequence. In embodiments, step c may comprise identifying whether one or more pairs of characteristic biological subsequences in the first processed biological sequence and one or more corresponding pairs of characteristic biological subsequences in the second processed biological sequence have a same or similar (e.g. differing by less than 100 units, preferably less than 50 units, yet more preferably less than 20 units, most preferably less than 10 units) inter-distance.

In embodiments, step c may further comprise comparing one or more second portions of the first processed biological sequence with one or more second portions in the second processed biological sequence. In embodiments, comparing one or more second portions may comprise comparing corresponding second portions (i.e. a second portion appearing in between a neighbouring pair of characteristic biological subsequences in the first processed biological sequence and a second portion appearing in between a corresponding neighbouring pair of characteristic biological subsequences in the first processed biological sequence).

In embodiments, step c may further comprise calculating a measure representing a degree of similarity (e.g. a Levenshtein distance) between the first and the second biological sequence.

In embodiments, the method may be used in a sequence similarity search, by comparing a query sequence with one or more other biological sequences (e.g. corresponding to a sequence database that is to be searched, for example in the form of a repository of processed biological sequences). In embodiments, a degree of similarity may be calculated for each of the other biological sequences. In embodiments, the method may comprise a further step of ranking the biological sequences (e.g. by decreasing degree of similarity). In embodiments, the method may comprise filtering the biological sequences. Filtering may be performed before and/or after step c. For example, filtering may be performed by selecting for comparison only those biological sequences from the database which fit a certain criterion, such as based on the organism or group of organisms which they derive from (e.g. plants, animals, humans, microorganisms, etc.), whether a secondary/tertiary/quaternary structure is known, their length, etc. Alternatively, filtering may be performed after the comparison has been performed, based on the same criteria or based on the calculated degree of similarity (e.g. only those sequences may be selected which surpass a certain threshold of similarity). In contrast to sequence similarity searching in the prior art, where an alignment step is typically required and a measure of similarity is then established therefrom, alignment is not strictly necessary for similarity searching according to embodiments. Indeed, similar sequences can already be found by simply searching for sequences with the same fingerprints (optionally also taking into account their order and their inter-distance), without alignment; this in turn allows to further speed up the search. The above notwithstanding, alignment in accordance with embodiments (cf. the eighth aspect) is also computationally simplified, so that it may be chosen to do an alignment anyway, even if not strictly required.

The method of this aspect thus allows determining (and optionally measuring) the similarity between a first and a second biological sequence. Such a comparison is also a cornerstone in other methods, such as those of the eighth and ninth aspect.

In an eighth aspect-considered useful for understanding the claimed invention-, a method for aligning a first biological sequence to a second biological sequence is described, comprising performing the method according to an embodiment of the seventh aspect, wherein step c further comprises aligning the fingerprint markers in the first processed biological sequence with the fingerprint markers in the second processed biological sequence. Fig. 5 schematically shows output results 400 from comparison unit 330 (which is in this case better referred to as 'alignment unit 330') in which biological sequences are aligned by their fingerprint markers.

Alignment in thus also simplified in embodiments, since a good alignment can already be obtained by simply aligning the fingerprints. Once more, this significantly reduces the computational complexity of the problem. Furthermore, in the prior art methods, such as those based on progressive alignment, there is a build-up of alignment errors, as misalignment for one of the earlier sequences typically propagate and cause additional misalignments in the later sequences. Conversely, since it is each time the same discrete set of fingerprint markers which are aligned (or at least attempted to) within one (multiple) alignment, there is no such propagation of errors.

In embodiments, the method may further comprise subsequently aligning corresponding second portions. Aligning the second portions may, for example, be performed using one of the alignment methods known in the prior art. Indeed, since the 'skeleton' of the alignment is already provided by aligning the fingerprint markers, only the alignment in between these markers is left to be fleshed out. Since each of these second portions is typically relatively short compared to the total biological sequence length, the known methods can typically perform such an alignment relatively quickly and efficiently.

In embodiments, the method may be for performing a multiple sequence alignment (i.e. the method may comprise aligning three or more biological sequences). In embodiments, the method may comprise aligning fingerprint markers in a third (or fourth, etc.) processed biological sequence with fingerprint markers in the first and/or second processed biological sequences. This is schematically depicted in Fig. 5 in which alignment unit 330 may also compare and align an arbitrary number of further processed biological sequences 213-216.

In embodiments, the method may be used in variant calling. In the case of sequence alignment between two biological sequences, the variant calling may identify variants (e.g. mutations) between a query sequence and a reference sequence. In the case of a multiple sequence alignment, the variant calling may identify the possible variations (which may include determining their frequency of occurrence) in a set of related sequences; optionally with respect to a reference sequence. Identifying variants may furthermore be performed on the basis of the primary structure, but may also take account of the secondary/tertiary/quaternary structure.

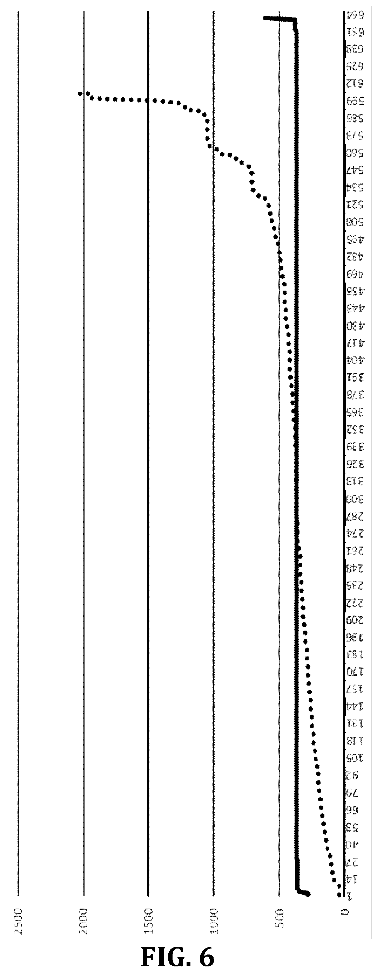

In a ninth aspect-in accordance with the claimed invention-, a method for performing a sequence assembly is described, comprising: (a) providing a first biological sequence, the first biological sequence being a biological sequence of a first biopolymer fragment, (b) providing a second biological sequence, the second biological sequence being either a biological sequence of a second biopolymer fragment or being a reference biological sequence, (c) aligning the first biological sequence to the second biological sequence using the method according any embodiment of the eighth aspect, and (d) merging the first biological sequence with the second biological sequence to obtain an assembled biological sequence. Fig. 6 schematically shows a sequence assembling unit 340 outputting assembled biological sequence 510, by first aligning (by their fingerprint markers) and subsequently merging an arbitrary number of biological sequences 500 (comprising of at least a first biological sequence 501 and second biological sequence 502).

In embodiments, the method steps a to d may be repeated so as to align and merge an arbitrary number of biopolymer fragments.

In order to facilitate sequencing, longer biopolymers can be fragmented, since the individual fragments are sequenced faster and more easily (e.g. they can be sequenced in parallel); as is known in the art. Sequence assembly is then typically used to align and merge fragment sequences to reconstruct the original sequence; this may also be referred to as 'read mapping', where 'reads' from a fragment sequence are 'mapped' to a second biopolymer sequence. Depending on the type of sequence assembly that is being performed, e.g. a de-novo assembly vs. a mapping assembly, the second biopolymer sequence may be selected to be a second biopolymer fragment or a reference sequence, as appropriate. Herein, a de-novo assembly is an assembly from scratch, without using a template (e.g. a backbone sequence). Conversely, a mapping assembly is an assembly by mapping one or more biopolymer fragment sequences to an existing backbone sequence (e.g. a reference sequence), which is typically similar (but not necessarily identical) to the to-be-reconstructed sequence. A reference sequence may for example be based on (part of) a complete genome or transcriptome, or may be have been obtained from an earlier de-novo assembly.

In embodiments, the method may comprise a further step (e), after step (d), of aligning the assembled biological sequence to the second biological sequence using the method according any embodiment of the eighth aspect. This additional alignment may be used to perform variant calling of the assembled biological sequence with respect to the second biological sequence (e.g. the reference sequence).

In a tenth aspect, a system comprising means for carrying out the method according to any embodiments of the second, third, fifth, seventh, eighth or ninth aspect is described.

The system may typically take on a different form depending on the method(s) it is meant to carry out. In embodiments, the system may be a sequencer (cf. second aspect), a sequence processing unit (cf. third aspect), a repository building unit (cf. fifth aspect), a comparison unit (cf. seventh aspect), an alignment unit (cf. eighth aspect), a sequence assembling unit (cf. ninth aspect). In embodiments, a generic data processing means (e.g. a personal computer or a smartphone) or a distributed computing environment (e.g. cloud-based system) can be configured to perform one or more of these functions. The distributed computing environment may, for example, comprise a server device and a networked client device. Herein, the server device may perform the bulk of one or more methods, including storing the repository of fingerprint data strings (cf. the first aspect) and the repository of processed biological sequences (cf. the sixth aspect). On the other hand, the networked client device may communicate instruction (e.g. input, such as a query sequence, and settings, such as search preferences) with the server device and may receive the method output. The above notwithstanding, the sequencer is typically a more dedicated device and may typically comprise further technical means for performing a sequencing. However, this does not exclude that the sequencer could be configured to also perform one or more further methods (e.g. a sequence assembly); in which case, the sequencer could for example also be referred to as an assembler. Similarly, the sequencer could be part of a distributed computing environment, where e.g. a client-side sequencing unit performs the physical sequencing and communicates with a cloud-based repository of fingerprint data strings.

In an eleventh aspect, a computer program product is described comprising instructions which, when the program is executed by a computer system, cause the computer system to carry out the method according to any embodiments of the second, third, fifth, seventh, eighth or ninth aspect.

In a twelfth aspect, a computer-readable medium is described comprising instructions which, when executed by a computer system, cause the computer system to carry out the method according to any embodiments of the second, third, fifth, seventh, eighth or ninth aspect.

In a thirteenth aspect-considered useful for understanding the claimed invention-, use of a library of biological sequence fingerprints as defined in any embodiment of the first aspect is described, for one or more selected from: sequencing a biopolymer or biopolymer fragment, performing a sequence assembly, processing a biological sequence, building a repository of processed biological sequences, comparing a first biological sequence to a second biological sequence, aligning a first biological sequence to a second biological sequence, performing a multiple sequence alignment, performing a sequence similarity search and performing a variant calling.

In a fourteenth aspect-considered useful for understanding the claimed invention-, use of a processed biological sequence as defined any embodiment of the fourth aspect or a library of processed biological sequences as defined in any embodiment of the sixth aspect is described, for one or more selected from: comparing a first biological sequence to a second biological sequence, aligning a first biological sequence to a second biological sequence, performing a multiple sequence alignment, performing a sequence similarity search and performing a variant calling.

In embodiments, any feature of any embodiment of any of the above aspects may independently be as correspondingly described for any embodiment of any of the other aspects.

Aspects of certain embodiments will now be described by a detailed description of several embodiments, the invention being limited only by the terms of the appended claims.

### Example 1-considered useful for understanding the claimed invention-:

### Comparison between a sequence search as known in the prior art and one using characteristic biological subsequences

### Example 1a: Using a short search string

Two separate searches were performed based on the search string "AVFPSIVGRPRHQGVMVGMGQKDSY". This corresponds to a relatively short protein sequence of length of 25 units, which could for example be a protein fragment in protein sequencing. Such a search could for example be used after sequencing of the fragment as part of identifying a suitable reference sequencing to use in a sequence assembly with the fragment.

The first search was performed using BLAST (Basic Local Alignment Search Tool); more particularly 'Protein BLAST' (available at the url: https://blast.ncbi.nlm.nih.gov/Blast.cgi?PROGRAM=blastp&PAGE TYPE=BlastSearch& LINK LOC=blasthome). The following search parameters were used: Database = Protein Data Bank proteins (pdb); Algorithm = blastp (protein-protein BLAST); Max target sequences = 1000; Short queries = Automatically adjust parameters for short input sequences; Expect threshold = 20000; Word size = 2; Matrix = PAM30; Compositional adjustment = No adjustment. BLAST required over 30 seconds for this search, after which 604 search results were returned.

On the other hand, based on the principles also underpinning the present invention, it was determined that "IVGRPRHQGVM" is a characteristic biological subsequence (i.e. a 'fingerprint') comprised in the above short protein sequence. As such, the second search was performed in a repository of processed biological sequences based on the search string "IVGRPRHQGVM". This repository was based on the same protein database as used in BLAST (i.e. Protein Data Bank; PDB), which had been previously processed using a repository of fingerprint data strings; i.e. characteristic biological subsequences represented by the fingerprint data strings were identified and marked in a set of publicly available biological sequences. This search returned 661 results. In contrast to BLAST, the time frame needed in this case was only 196 milliseconds. As such, even for such a relatively short sequence, it was observed that the present method was able to reduce the required time by a factor of over 150 compared to the known-art method.

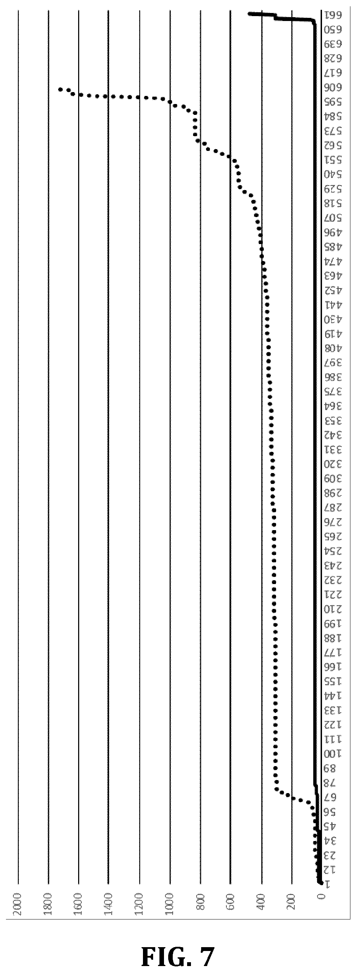

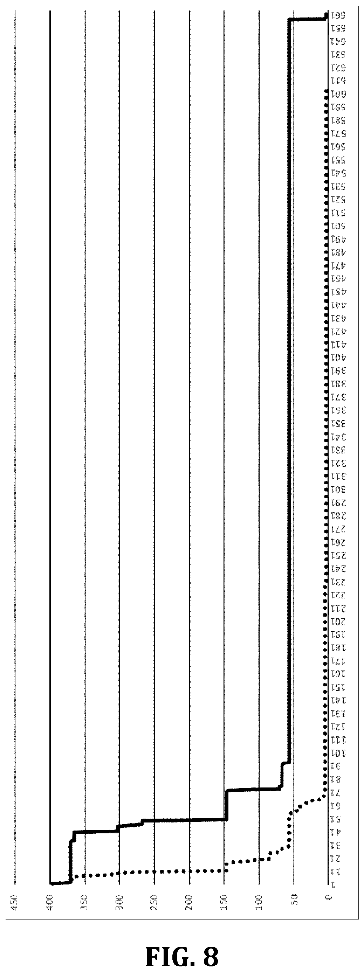

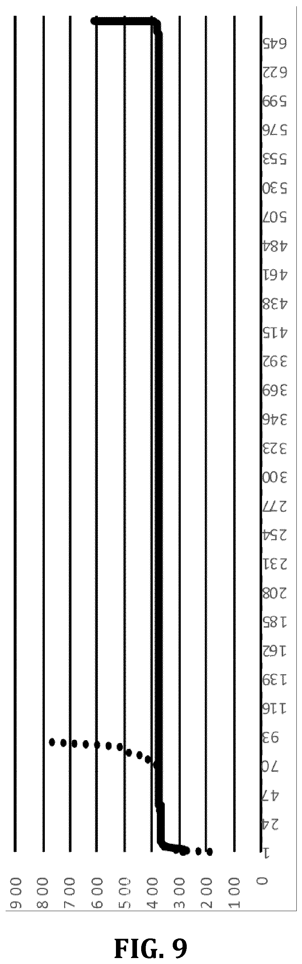

We now refer to Figs. 7, 8 and 9, showing the results of both of these searches (BLAST = dotted line; present method = solid line) in terms of their total length (Fig. 7), their Levenshtein distance (Fig. 8) and longest common substring (Fig. 9). For each graph, the search results are shown ordered from low to high with respect to the plotted parameter (i.e. total length, Levenshtein distance or longest common substring). Furthermore, one of the search result, namely the protein sequence 5NW4_V (i.e. the first result listed by BLAST), was selected as a reference with respect to which the Levenshtein distance and the longest common substring were calculated. As can be observed in these figures, the present method yielded, across the full range of search results, a smaller variation in total length (characterized by a relative plateau spanning over a significant portion of the results), a considerably lower Levenshtein distance and a considerably larger longest common substring; compared to the BLAST results. The combination of these suggests that the method using characteristic biological subsequences was able to identify results which are more relevant for the performed search.

### Example 1b: Using a longer protein as the search string

The previous example was repeated, but this time a complete protein sequence, 3MN5_A (with a length of 359 units), was searched.

The first search, using BLAST, returned 88 search results.

On the other hand, based on the principles also underpinning the present invention, it was determined that six characteristic biological subsequences (i.e. 'fingerprints') could be found in the sequence 3MN5_A; these were denoted as:
+4641474444415052415646_1, +495647525052485147564d_1,
+4949544e5744444d454b49_1, +494d464554464e5650414d_1,
+494b454b4c435956414c44_1 and +49474d4553414749484554_1,
where e.g. '49474d4553414749484554' corresponds to the respective subsequence in hexadecimal format. As such, the second search was performed, in the same repository of processed biological sequences as in the previous example, to find those protein sequences which comprise the same six characteristic biological subsequences in the same order. This search returned 661 results.

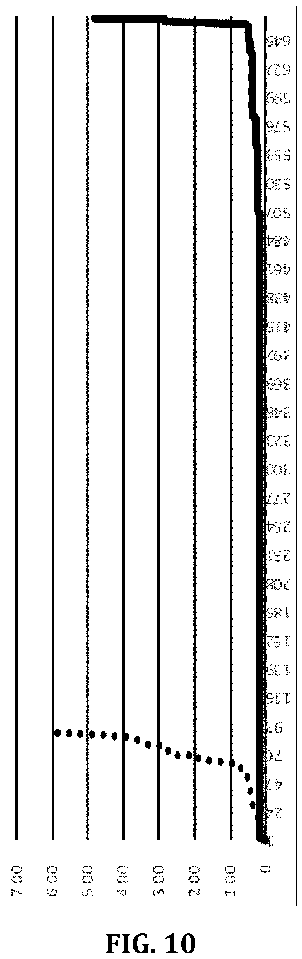

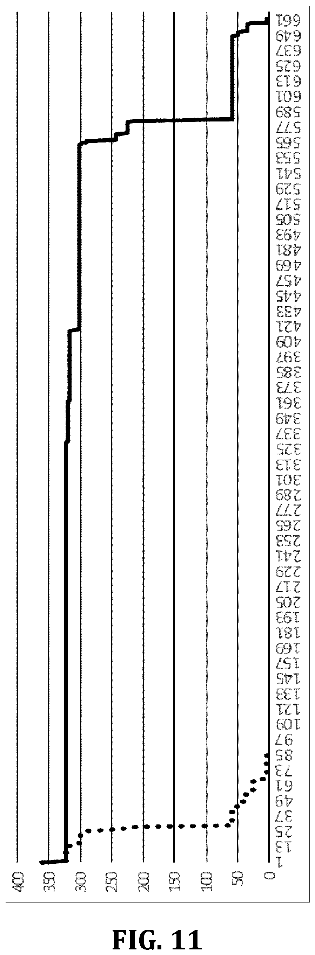

We now refer to Fig. 10, 11 and 12, showing the results of both of these searches (BLAST = dotted line; present method = solid line) in terms of their total length (Fig. 10), their Levenshtein distance (Fig. 11) and longest common substring (Fig. 12). For each graph, the search results are shown ordered from low to high with respect to the plotted parameter (i.e. total length, Levenshtein distance or longest common substring). In this case, the Levenshtein distance and the longest common substring were calculated with respect to the original query sequence 3MN5_A. As can be observed in these figures, the characteristics of the search results for both methods are relatively comparable at the extremes. However, the present method yielded in the intermediate range a plateau of results with little variation in total length, a low Levenshtein distance and a fairly high longest common substring. The combination of these suggests that the method using characteristic biological subsequences was able to identify a larger number of relevant results.

## Claims

1. A computer-implemented method for sequencing a biopolymer or biopolymer fragment (500), taking into account information contained in a repository of fingerprint data strings (100), each fingerprint data string representing a characteristic biological subsequence, each characteristic biological subsequence being **characterized by** having for the unit directly following or preceding it less possible options than the maximum number of different units, the unit being an amino acid when the characteristic biological subsequence relates to a protein and a codon when the characteristic biological subsequence relates to DNA or RNA,
the repository comprising at least
- a first fingerprint data string representing a first characteristic biological sequence of a first length,
- a second fingerprint data string representing a second characteristic biological subsequence of a second length, and
- for at least one of the fingerprint data strings, combinatory data related to one or more units which can appear directly before or after the characteristic biological subsequence when said characteristic biological subsequence is present in a biological sequence,
wherein the first and the second length are equal to 4 or more and wherein the first and the second length differ from one another;
the method comprising:
a. sequencing a provisional biological sequence for said biopolymer or biopolymer fragment, and
b. processing the provisional biological sequence by the computer-implemented steps of:
b1. searching the provisional biological sequence for occurrences of one or more of the characteristic biological subsequences represented by the fingerprint data strings and subsequently
b2. validating or rejecting the provisional biological sequence by, for each occurrence, determining whether or not a unit appearing directly before or after the characteristic biological subsequence conforms with the combinatory data in the repository,
and/or
b1'. searching a head or tail of the partial biological sequence for occurrences of one of the characteristic biological subsequences represented by the fingerprint data strings and subsequently
b2'. predicting a unit appearing directly before or after the characteristic biological subsequence from the combinatory data in the repository.

2. The method according to claim 1, wherein step b2 further comprises determining whether the provisional biological subsequence conforms with data related to a secondary and/or tertiary and/or quaternary structure of the characteristic biological subsequence when said characteristic biological subsequence is present in a biopolymer.

3. The method according to any of the previous claims, wherein step b2' further comprises predicting the unit from data related to a secondary and/or tertiary and/or quaternary structure of the characteristic biological subsequence when said characteristic biological subsequence is present in a biopolymer.

4. The method according to any of the previous claims, wherein step b2' further comprises predicting the unit from data related to a relationship between the characteristic biological subsequence and one or more further characteristic biological subsequences.

5. A computer-implemented method for performing a sequence assembly, comprising:
a. providing a first biological sequence (501), the first biological sequence being a biological sequence of a first biopolymer fragment,
b. providing a second biological sequence (502), the second biological sequence being either a biological sequence of a second biopolymer fragment or being a reference biological sequence,
c. aligning the first biological sequence (501) to the second biological sequence (502) by aligning fingerprint markers in the first biological sequence with fingerprint markers in the second biological sequence, the fingerprint markers being associated with fingerprint data strings representing characteristic biological subsequences, each characteristic biological subsequence being **characterized by** having for the unit directly following or preceding it less possible options than the maximum number of different units, the unit being an amino acid when the characteristic biological subsequence relates to a protein and a codon when the characteristic biological subsequence relates to DNA or RNA, and
d. merging the first biological sequence with the second biological sequence to obtain an assembled biological sequence (510).

6. A system (310, 320, 330, 340, 350) comprising means for carrying out the method according to any of the previous claims.

7. A system according to claim 6, the system being an assembler.

8. A computer program product comprising instructions which, when the program is executed by a computer system, cause the computer system to carry out the method according to any of claims 1 to 5.

9. A computer-readable medium comprising instructions which, when executed by a computer system, cause the computer system to carry out the method according to any of claims 1 to 5.

## Patentansprüche

1. Ein computerimplementiertes Verfahren zum Sequenzieren eines Biopolymers oder Biopolymerfragments (500) unter Berücksichtigung von Informationen, die in einem Archiv von Fingerabdruckdatensträngen (100) enthalten sind, wobei jeder Fingerabdruckdatenstrang eine charakteristische biologische Teilsequenz darstellt, wobei jede charakteristische biologische Teilsequenz **dadurch gekennzeichnet ist, dass** sie für die ihr direkt folgende oder vorangehende Einheit weniger mögliche Optionen aufweist, als die maximale Anzahl unterschiedlicher Einheiten, wobei die Einheit eine Aminosäure ist, wenn sich die charakteristische biologische Teilsequenz auf ein Protein bezieht, und ein Codon, wenn sich die charakteristische biologische Teilsequenz auf DNA oder RNA bezieht, wobei das Archiv mindestens umfasst
- einen ersten Fingerabdruckdatenstrang, der eine erste charakteristische biologische Sequenz mit einer ersten Länge darstellt,
- einen zweiten Fingerabdruckdatenstrang, der eine zweite charakteristische biologische Teilsequenz mit einer zweiten Länge darstellt, und
- für mindestens eine der Fingerabdruckdatenstränge kombinatorische Daten, die sich auf eine oder mehrere Einheiten beziehen, die direkt vor oder nach der charakteristischen biologischen Teilsequenz erscheinen können, wenn die charakteristische biologische Teilsequenz in einer biologischen Sequenz vorhanden ist,
wobei die erste und die zweite Länge gleich 4 oder mehr sind, und wobei sich die erste und die zweite Länge voneinander unterscheiden;
wobei das Verfahren umfasst:
a. Sequenzieren einer vorläufigen biologischen Sequenz für das Biopolymer oder Biopolymerfragment, und
b. Verarbeiten der vorläufigen biologischen Sequenz durch die computerimplementierten Schritte von:
b1. Durchsuchen der vorläufigen biologischen Sequenz nach Vorkommen einer oder mehrerer der charakteristischen biologischen Teilsequenzen, die von den Fingerabdruckdatensträngen dargestellt sind, und danach
b2. Validieren oder Ablehnen der vorläufigen biologischen Sequenz durch, bei jedem Vorkommen, Bestimmen, ob eine Einheit, die direkt vor
oder nach der charakteristischen biologischen Teilsequenz erscheint, mit den kombinatorischen Daten im Archiv übereinstimmt oder nicht, und/oder
b1'. Durchsuchen eines Kopfes oder Endes der biologischen Teilsequenz nach Vorkommen einer der charakteristischen biologischen Teilsequenzen, die von den Fingerabdruckdatensträngen dargestellt sind, und danach
b2'. Vorhersagen einer Einheit, die direkt vor oder nach der charakteristischen biologischen Teilsequenz erscheint, aus den kombinatorischen Daten im Archiv.

2. Das Verfahren nach Anspruch 1, wobei Schritt b2 weiter Bestimmen umfasst, ob die vorläufige biologische Teilsequenz mit Daten übereinstimmt, die sich auf eine sekundäre und/oder tertiäre und/oder quartäre Struktur der charakteristischen biologischen Teilsequenz beziehen, wenn die charakteristische biologische Teilsequenz in einem Biopolymer vorhanden ist.

3. Das Verfahren nach einem der vorstehenden Ansprüche, wobei Schritt b2' weiter Vorhersagen der Einheit aus Daten umfasst, die sich auf eine sekundäre und/oder tertiäre und/oder quartäre Struktur der charakteristischen biologischen Teilsequenz beziehen, wenn die charakteristische biologische Teilsequenz in einem Biopolymer vorhanden ist.

4. Das Verfahren nach einem der vorstehenden Ansprüche, wobei Schritt b2' weiter Vorhersagen der Einheit aus Daten umfasst, die sich auf eine Beziehung zwischen der charakteristischen biologischen Teilsequenz und einer oder mehreren weiteren charakteristischen biologischen Teilsequenzen beziehen.

5. Ein computerimplementiertes Verfahren zum Durchführen einer Sequenzzusammensetzung, umfassend:
a. Bereitstellen einer ersten biologischen Sequenz (501), wobei die erste biologische Sequenz eine biologische Sequenz eines ersten Biopolymerfragments ist,
b. Bereitstellen einer zweiten biologischen Sequenz (502), wobei die zweite biologische Sequenz entweder eine biologische Sequenz eines zweiten Biopolymerfragments ist oder eine biologische Referenzsequenz ist,
c. Ausrichten der ersten biologischen Sequenz (501) an der zweiten biologischen Sequenz (502) durch Ausrichten von Fingerabdruckmarkierungen in der ersten biologischen Sequenz mit Fingerabdruckmarkierungen in der zweiten biologischen Sequenz, wobei die Fingerabdruckmarkierungen mit Fingerabdruckdatensträngen verknüpft sind, die
charakteristische biologische Teilsequenzen darstellen, wobei jede charakteristische biologische Teilsequenz **dadurch gekennzeichnet ist, dass** sie für die ihr direkt folgende oder vorangehende Einheit weniger mögliche Optionen aufweist, als die maximale Anzahl unterschiedlicher Einheiten, wobei die Einheit eine Aminosäure ist, wenn sich die charakteristische biologische Teilsequenz auf ein Protein bezieht, und ein Codon, wenn sich die charakteristische biologische Teilsequenz auf DNA oder RNA bezieht, und
d. Zusammenführen der ersten biologischen Sequenz mit der zweiten biologischen Sequenz, um eine zusammengesetzte biologische Sequenz zu erhalten (510).

6. Ein System (310, 320, 330, 340, 350), das Mittel zum Ausführen des Verfahrens nach einem der vorstehenden Ansprüche umfasst.

7. Ein System nach Anspruch 6, wobei das System ein Assembler ist.

8. Ein Computerprogrammprodukt, das Anweisungen umfasst, die, wenn das Programm von einem Computersystem ausgeführt wird, das Computersystem veranlassen, das Verfahren nach einem der Ansprüche 1 bis 5 durchzuführen.

9. Ein computerlesbares Medium, das Anweisungen umfasst, die, wenn sie von einem Computersystem ausgeführt werden, das Computersystem veranlassen, das Verfahren nach einem der Ansprüche 1 bis 5 durchzuführen.

## Revendications

1. Un procédé mis en œuvre par ordinateur pour séquencer un biopolymère ou un fragment de biopolymère (500), en tenant compte des informations contenues dans un dépôt de chaînes de données d'empreintes digitales (100), chaque chaîne de données d'empreintes digitales représentant une sous-séquence biologique caractéristique, chaque sous-séquence biologique caractéristique étant **caractérisée par le fait que** l'unité la suivant ou la précédant directement a moins d'options possibles que le nombre maximum d'unités différentes, l'unité étant un acide aminé lorsque la sous-séquence biologique caractéristique se rapporte à une protéine et un codon lorsque la sous-séquence biologique caractéristique se rapporte à l'ADN ou à l'ARN, le dépôt comprenant au moins
- une première chaîne de données d'empreintes digitales représentant une première séquence biologique caractéristique d'une première longueur,
- une deuxième chaîne de données d'empreintes digitales représentant une deuxième sous-séquence biologique caractéristique d'une deuxième longueur, et
- pour au moins une des chaînes de données d'empreintes digitales, des données combinatoires relatives à une ou plusieurs unités pouvant apparaître directement avant ou après la sous-séquence biologique caractéristique lorsque ladite sous-séquence biologique caractéristique est présente dans une séquence biologique,
dans lequel la première et la deuxième longueur sont égales à 4 ou plus et dans lequel la première et la deuxième longueur diffèrent l'une de l'autre ;
le procédé comprenant :
a. séquencer une séquence biologique provisoire pour ledit biopolymère ou fragment de biopolymère, et
b. traiter la séquence biologique provisoire par les étapes mises en œuvre par ordinateur suivantes :
b1.rechercher dans la séquence biologique provisoire des occurrences d'une ou plusieurs des sous-séquences biologiques caractéristiques représentées par les chaînes de données d'empreintes digitales et ensuite
b2. valider ou rejeter la séquence biologique provisoire en déterminant, pour chaque occurrence, si une unité apparaissant directement avant ou après la sous-séquence biologique caractéristique est conforme aux données combinatoires dans le dépôt,
et/ou
b1'.rechercher une tête ou une queue de la séquence biologique partielle pour des occurrences d'une des sous-séquences biologiques caractéristiques représentées par les chaînes de données d'empreintes digitales et ensuite
b2'. prédire une unité apparaissant directement avant ou après la sous-séquence biologique caractéristique à partir des données combinatoires dans le dépôt.

2. Le procédé selon la revendication 1, dans lequel l'étape b2 comprend en outre la détermination de la conformité de la sous-séquence biologique provisoire avec des données relatives à une structure secondaire et/ou tertiaire et/ou quaternaire de la sous-séquence biologique caractéristique lorsque ladite sous-séquence biologique caractéristique est présente dans un biopolymère.

3. Le procédé selon l'une quelconque des revendications précédentes, dans lequel l'étape b2' comprend en outre la prédiction de l'unité à partir de données relatives à une structure secondaire et/ou tertiaire et/ou quaternaire de la sous-séquence biologique caractéristique lorsque ladite sous-séquence biologique caractéristique est présente dans un biopolymère.

4. Le procédé selon l'une quelconque des revendications précédentes, dans lequel l'étape b2' comprend en outre la prédiction de l'unité à partir de données relatives à une relation entre la sous-séquence biologique caractéristique et une ou plusieurs autres sous-séquences biologiques caractéristiques.

5. Un procédé mis en œuvre par ordinateur pour effectuer un assemblage de séquence, comprenant :
a. fournir une première séquence biologique (501), la première séquence biologique étant une séquence biologique d'un premier fragment de biopolymère,
b. fournir une deuxième séquence biologique (502), la deuxième séquence biologique étant soit une séquence biologique d'un deuxième fragment de biopolymère, soit une séquence biologique de référence,
c. aligner la première séquence biologique (501) à la deuxième séquence biologique (502) en alignant des marqueurs d'empreintes digitales dans la première séquence biologique avec des marqueurs d'empreintes digitales dans la deuxième séquence biologique, les marqueurs d'empreintes digitales étant associés à des chaînes de données d'empreintes digitales représentant des sous-séquences biologiques caractéristiques, chaque sous-séquence biologique caractéristique étant **caractérisée par le fait que** l'unité la suivant ou la précédant directement a moins d'options possibles que le nombre maximum d'unités différentes, l'unité étant un acide aminé lorsque la sous-séquence biologique caractéristique se rapporte à une protéine et un codon lorsque la sous-séquence biologique caractéristique se rapporte à l'ADN ou à l'ARN, et
d. fusionner la première séquence biologique avec la deuxième séquence biologique pour obtenir une séquence biologique assemblée (510).

6. Un système (310, 320, 330, 340, 350) comprenant des moyens pour réaliser le procédé selon l'une quelconque des revendications précédentes.

7. Un système selon la revendication 6, le système étant un assembleur.

8. Un produit de programme informatique comprenant des instructions qui, lorsque le programme est exécuté par un système informatique, amènent le système informatique à réaliser le procédé selon l'une quelconque des revendications 1 à 5.

9. Un support lisible par ordinateur comprenant des instructions qui, lorsqu'elles sont exécutées par un système informatique, amènent le système informatique à réaliser le procédé selon l'une quelconque des revendications 1 à 5.
